Europäisches Patentamt

(19) European Patent Office

Office européen des brevets

(11) Publication number: **0 204 438 B1**

## (12) EUROPEAN PATENT SPECIFICATION

(45) Date of publication of patent specification :
06.03.91 Bulletin 91/10

(51) Int. Cl.⁵ : **C12Q 1/58,** // G01N33/52,
C12Q1/04

(21) Application number : 86303493.0

(22) Date of filing : 08.05.86

(54) Compositions and methods for the detection of urease for the diagnosis of campylobacter pyloridis infection.

(30) Priority : 17.05.85 AU 611/85
13.06.85 US 744840

(43) Date of publication of application :
10.12.86 Bulletin 86/50

(45) Publication of the grant of the patent :
06.03.91 Bulletin 91/10

(84) Designated Contracting States :
AT BE CH DE FR GB IT LI LU NL SE

(56) References cited :
EP-A- 0 018 825
FR-A- 2 442 268
GB-A- 1 112 251
US-A- 3 145 086
US-A- 4 101 382
US-A- 4 282 316

(56) References cited :
PATENTS ABSTRACTS OF JAPAN, vol. 7, no. 172 (P-213)[1317], 29th July 1983; & JP-A-58 77 663 (KIYOUTO DAIICHI KAGAKU K.K.)
ANALYTICAL CHEMISTRY, vol. 51, no. 2, February 1979, pages 199-203, Columbus, Ohio, US; J. RUZICKA et al.: "Enzymatic determination of urea in serum based on pH measurement with the flow Injection method"

(73) Proprietor : Marshall, Barry James, M.D.
25 Bondi Street
Mount Hawthorn 6016 Perth Western Australia (AU)

(72) Inventor : Marshall, Barry James, M.D.
25 Bondi Street
Mount Hawthorn 6016 Perth Western Australia (AU)

(74) Representative : Lyons, Andrew John et al
ROYSTONS 531 Tower Building Water Street
Liverpool L3 1BA (GB)

EP 0 204 438 B1

**Description**

BACKGROUND OF THE INVENTION

This invention relates to compositions and methods for the detection of urease as a means of diagnosing the presence of gastrointestinal disease.

Factors adversely affecting the function of the gastrointestinal system in humans are exceedingly varied in their nature. Such disorders may arise in the upper or lower gastrointestinal tracts or both. There is a broad range of causes of gastrointestinal disorders, including genetic, physiological, environmental, and psychogenic factors. Accordingly, the diagnosis and management of these disorders can be exceptionally difficult. A detailed discussion of gastrointestinal tract functions, disorders, causes, and treatments can be found in Spiro, Clinical Gastroenterology (3d. edition 1983).

Among the chronic disorders of the upper gastrointestinal tract are those which fall under the general categories of gastritis and peptic ulcer disease. (The upper gastrointestinal tract is generally defined as including the esophagus, the stomach, the duodenum, the jejunum, and ilium.) Peptic ulcers are lesions of the gastrointestinal tract lining, characterized by loss of tissue due to the action of digestive acids and pepsin. It has been generally held that peptic ulcers are caused either by gastric hypersecretion, or (more often) by decreased resistance of the gastric lining to digestive acids and pepsin. Gastritis is, by definition, typified by an inflammation of the stomach mucosa. In practice, though, the disorder is manifested by a broad range of poorly-defined, and heretofore inadequately treated, symptoms such as indigestion, "heart burn", dyspepsia and excessive eructation. A general discussion of gastritis appears in B.J. Marshall and J.R. Warren, "Unidentified Curved Bacilli in the Stomach of Patients with Gastritis and Peptic Ulceration". The Lancet, 1311-1315 (1984), and in R.Greenlaw, et al., "Gastroduodenitis, A Broader Concept of Peptic Ulcer Disease". 25 Digestive Diseases and Sciences 660 672 (1980).

As with the management of any disorder, the rapid precise, and accurate diagnosis of gastrointestinal disorders is of paramount importance. However, the diagnostic methods typically employed in the art are often slow, cumbersome, costly and may yield equivocal or inaccurate results. See, e.g., Spiro, supra.

It has been discovered that many disorders affecting the upper gastrointestinal tract are mediated by bacteria, such as those of the genus Campylobacter, particularly Campylobacter pyloridis.

Presently used methods of detecting campylobacter pyloridis are time consuming and expensive. In particular, it is not possible to complete a test to detect the infection within the time of an appointment of a patient with a physician.

Thus, there is a need for further consultation.

Campylobacter pyloridis is almost unique in its ability to produce large amounts of urease enzyme. Urease splits urea to form ammonia and carbon dioxide.

It has now been discovered that the urease enzyme produced by campylobacter pyloridis is so active that it destroys all urea present in gastric juices of humans.

The present invention provides a method for the detection of preformed urease present in gastric tissue infected with campylobacter pyloridis. The presence of such preformed urease in gastric mucosa is pathognomonic of the infection and indicates that gastritis is present.

Further, the method of the present invention has general applicability for the detection of preformed urease.

For example, it may be used for the detection of preformed urease in the vomitus of patients with campylobacter pyloridis gastritis. Also, it may be used to detect the presence of preformed urease in leguminous plants. The presence of urease in these plants may be an indication of the presence of toxins in pasture which can cause gas production or bloat in animals.

The methods and compositions of this invention thus provide a rapid, inexpensive, and accurate diagnosis of the presence of urease resulting from such disorders.

SUMMARY OF THE INVENTION

In accordance with one aspect of the present invention there is provided a composition for the diagnosis of gastrointestinal disorder in a human or lower animal subject by detection of urease in gastric material of the subject, which comprises

(a) urea, preferably at a concentration of from about 10 to about 40 grams per litre ;

(b) a bactericide which substantially inhibits growth of urease producing organisms, preferably at a concentration of from about 1 to about 5 grams per litre ;

(c) a pH indicator which undergoes a colour change upon an increase of pH, at an effective concentration ; and

(d) water ;

wherein said composition has an acid pH of at least 5.0, and the pH of said composition is at least about one pH unit lower then the pKa of said indicator.

Preferably, the composition of the present invention is in gel form, containing a gelling agent at a concentration of from about 5 to about 50 grams per litre. In accordance with another aspect of the present invention there is provided a method for the diagnosis of gastrointestinal disorder in a human or lower animal subject by detection of urease in gastric material of the subject, comprising the steps of obtaining a sample of gastric material from said subject, contacting said sample with a composition of the present invention, and observing any colour change in said composition.

All concentrations herein are by weight of component per volume of total composition.

## DESCRIPTION OF THE DRAWINGS

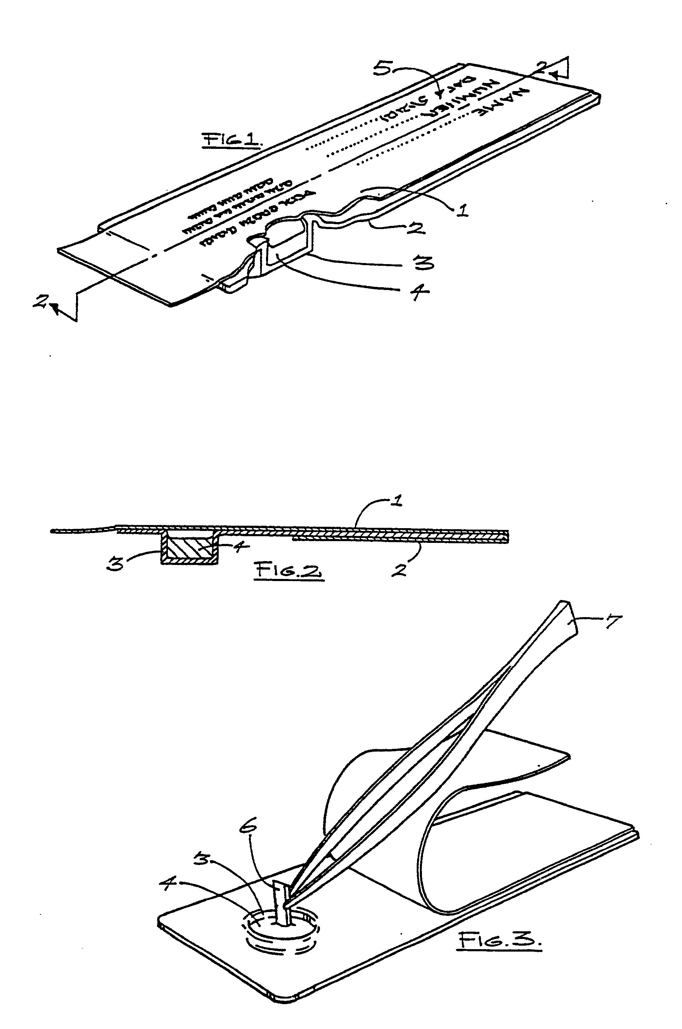

The accompanying drawings are of a preferred test device useful in the method of the present invention. In particular, Figure 1 is an isometric view of a test device of this invention, with a cutaway exposing a container containing a composition of this invention ;

Figure 2 is a sectional view of the device of Figure 1 ; and

Figure 3 is an isometric view of the device of Figure 1, showing the device in use.

## DESCRIPTION OF THE INVENTION

As used herein, "an effective concentration" of indicator is a concentration of indicator which effects a readily-discernible colour of the composition of this invention when used according to the method of this invention. Typically, the indicator is present at a level of from about 2 to about 150 milligrams per litre, preferably from about 20 to about 150 milligrams per litre. It is found that amounts of urea in the range from about 10 to about 40 grams per litre of the composition of the present invention are sufficient for reaction with the preformed urease present in typical specimens to produce a colour change. When the urease contacts the urea a reaction follows which produces ammonia.

The presence of ammonia increases the pH of the adjacent portion of the carrier medium and if sufficient ammonia is produced there is a resultant colour change because of the presence of the pH indicator.

More preferably, the composition of the present invention contains urea at a concentration of from about 20 to about 40 grams per litre.

As used herein, "gastrointestinal disorder" encompasses any disease or other disorder of the gastrointestinal tract of a human or lower animal. Such gastrointestinal disorders include, for example : disorders not manifested by presence of ulcerations in the gastric mucosa (herein "non-ulcerative gastrointestinal disorder"), including chronic or atrophic gastritis, gastroenteritis, non-ulcer dyspepsia, esophogeal reflux disease and gastric motility disorders ; and "peptic ulcer disease", i.e., gastric and duodenal ulcers. In particular, "gastrointestinal disorder" refers to such disorders of the upper gastrointestinal tract caused or mediated by bacteria, including campylobacter-like organisms (herein "CLO"), e.g., Campylobacter pyloridis. Such CLO include those described in J.R. Warren and B.J. Marshall, "Unidentified Curved Bacilli on Gastric Epithelium in Active Chronic Gastritis", The Lancet 1273-1275 (1983).

Urea is of the formula $H_2NCONH_2$, and is a naturally occurring product of protein metabolism. Urea for use in the compositions of this invention is available from a variety of commercial sources. As a basis for this invention, it has been found that gastric materials from humans or other animals having gastrointestinal disorders contain relatively large quantities of urease (urea amidohydrolase), which hydrolyses urea to ammonia and carbon dioxide (or ammonium carbonate). The compositions of this invention serve, in part, to detect the presence of urease through its hydrolysis of urea.

The pH indicators useful in this invention are weak acids, with sharply different colours in their dissociated (ionized) and undissociated (neutral) states. The indicators useful herein preferably have $pK_a$ values of from about 6.5 to about 8.5, preferably from about 7.0 to about 8.0. The colour exhibited by the indicator in the present composition will depend upon the pH of the composition, the particular indicator used, and the dissociation constant ($K_a$) for that indicator (i.e., $pK_a = -\log_{10} K_a$). As the colour exhibited by the indicator changes over a range of pH values (pH $= -\log_{10} [H^+]$), the indicators useful in the present composition change colour over a pH range of from about 5.5 to about 9.0, preferably from about 6.5 to about 8.5. The pH of the composition of the present invention is, accordingly, adjusted to a pH at least about one pH unit lower than the $pK_a$ of the indicator used (i.e., having a hydrogen ion concentration [H⁺] ten times less than (10% of) the hydrogen ion concentration in a solution having a pH equal to the $pK_a$ of the indicator).

Preferably, the pH is adjusted to a pH about two pH units below the $pK_a$ of the indicator. Adjustment of the pH of the present compositions can be effected by addition of a base (e.g., sodium hydroxide) or an acid (e.g., hydrochloric acid or citric acid). Thus, preferably, the pH of the composition of this invention is adjusted to a pH of from about 5.0 to about 6.5, more preferably from about 5.0 to about 6.0.

Indicators among those useful herein include p-nitrophenol, bromothymol blue (dibromothymolsulfonphthalein), phenol red (phenolsulfonphthalein), neutral red (2-methyl-3-amino-6-dimethylaminophenazine), quinoline blue (cyanine), cresol red (o-cresolsulfonphthalein), metacresol purple (m-cresolsulfonphthalein), and thymol blue (thymolsulfonphthalein). Bromothymol blue, phenol red, neutral red and cresol red are preferred indicators for use in the compositions of this invention.

Indicators among those useful herein are described in the The Merck Index (9th ed. 1976).

The bactericide incorporated in the compositions of this invention is one or more materials which substantially inhibit the growth of urease-producing organisms in the composition. The bactericide is most preferably present in an amount in the range from 2 to 4 grams per litre of the composition.

Bactericides useful in this invention include sodium azide and methylhydroxybenzoate. Methylhydroxybenzoate is a particularly preferred bactericide. The specific amount of bactericide to be used in this present composition depends upon factors well known in the microbiological arts, such as the particular bactericide used, and the bactericidal properties (if any) of the other components in the present compositions. The presence of the bactericide means that in the absence of an excess of alkali the test will only give a positive result when there is preformed urease present in the sample of gastric material.

The compositions of this invention may contain optional components which affect the performance or physical characteristics of the compositions. Such additional components must not, however, interfere with the indicator, as by obscuring the colours exhibited by the indicator.

The compositions of this invention preferably contain a gelling agent, so that the compositions are in a semisolid state at ambient conditions. A particularly preferred gelling agent is agar, preferably present at a level of from about 5 to about 50 grams per litre, more preferably from about 10 to about 20 grams per litre. The agar used in the compositions of this invention is readily available from a variety of commercial sources. Typically, agar (a polysaccharide complex) is extracted from the agarocytes of certain algae. Also, preferably, the agar used in the present composition is nonnutritive, i.e., does not support the growth of microorganisms In fact since the test of the present invention is intended to detect only the presence of preformed bacterial urease, the usual microbial nutrients may be omitted from the compositions.

A particularly preferred optional component of the present invention is a buffer. As stated above, the compositions of this invention are adjusted to a pH at least one pH unit below the $pK_a$ of the indicator used. Thus, preferably, the pH of the present composition is from about 5.0 to about 6.5, more preferably from about 5.0 to about 6.0. This pH of the final composition is preferably effected by the addition of a suitable buffer. Such buffers are well known in the chemical art, including the use of such weak acid salts as sodium bisulfate, sodium acetate and sodium phosphate.

The total amount of buffer incorporated in the present composition will depend upon the total amount of urea incorporated in the composition, such that the buffer does not prevent sufficient change in composition pH (resulting from hydrolysis of the urea present), so as to cause a change in the colour of the indicator used.

However, the buffer is preferably present in a concentration sufficient to prevent substantial changes in composition pH, and (thereby) spurious indicator colour changes, due to chemicals other than preformed urease enzyme in the gastric material sample to be analyzed. Typically, then, the buffer is incorporated in the present composition at concentrations of from about 50 to about 2000 milligrams per litre. (As used herein, the buffer concentration includes the concentration of the buffer salt and of the acid used to effect pH adjustment of the composition.)

As used herein, "gastric material" refers to any material obtained directly or indirectly from the upper gastrointestinal tract of a human or other animal. Such materials include, for example, gastric epithelium, gastric mucosa, and digestive fluids. Samples of such materials, for use in the method of this invention, may be obtained by any of a variety of well known methods, according to sound medical practice. Such methods include, for example, obtaining the sample by biopsy of the subject, obtaining the sample from the vomitus of the subject, and obtaining the sample from nasal gastric aspirate.

As used herein, "contacting" the sample with the composition of the present invention, refers to any method which effects substantial interface between a composition of this invention and the sample gastric material, for a time sufficiently long so as to allow the hydrolysis of urea by any urease present in the sample to an extent sufficient to produce a detectable colour change. Such time is typically longer than about five minutes and is typically in the range from 5 to 15 minutes. Preferably, though, the gastric material sample is immersed, or substantially immersed, in a composition of this invention. Also, preferably, care is taken

4

so as to avoid contamination of the gastric material sample with organisms from a source other than the stomach of the subject to be diagnosed.

In the event that the gastric material used constitutes digestive fluids, then a preferred optional step in the present processes is testing the pH of said gastric material. Preferably, then, the sample is contacted with a pH-test composition which is an aqueous solution of the particular indicator used in said composition of this invention (without urea), adjusted to the same pH as said urea-containing composition of this invention. If the gastric material effects no change in the indicator colour of the pH-test composition, then the material is of an acid pH, and may be used directly in the contacting step of the process of this invention described above. If, however, the colour of the pH-test composition changes, then the pH of the gastric material should be acidified, as by addition of an acid.

The observing step of this process entails detection of any colour change in the composition colour, to the colour exhibited by the indicator in its dissociated state. Failure of the composition to change colour after about twenty-four hours reflects a negative test result.

In most positive cases the physician will have the results available before the patient leaves, and he may, therefore, prescribe therapy immediately. Further tests may not be required. When small numbers of bacteria are present a delayed reaction may occur which can be read the next day but this occurs in less than 10% of positive cases. As all patients infected with campylobacter pyloridis have gastritis a positive result also indicates the presence of this condition. Also, as indicated above, the present invention may be extended to veterinary use such as testing of pasture to ensure that it is at the right stage of maturity for grazing and does not contain excessive quantities of plant urease.

The method of this invention is preferably performed using a device, herein "diagnostic device", which contains a quantity of a composition of this invention in an easily-handled container. Such devices may comprise :

(a) a container, having an opening aperture area of from about 20 square millimetres to about 200 square millimetres and having a total contained volume of from about 40 cubic millimetres to about 1000 cubic millimetres ; and

(b) a cover for said container, affixed to said container by a means which allows said cover to be moved so as to open and close said container opening ;

wherein said container contains from about 0.04 to about 2.0 millilitres of a composition of this invention.

Preferably, the container contains from about 0.20 millilitres to about 0.40 millilitres of a composition of this invention.

One such preferred diagnostic device is pictured in Figures 1 through 3 of the Drawings. Figure 1 and Figure 2 show a container 3 which is formed as a concave form, or well, in a continuous, flat holder 2, which is a sheet of rigid, or semi-rigid, water-impermeable material. The container contains a composition of this invention 4. A cover 1, is affixed to the container, and made of a flexible, water-impermeable material, and is of the same dimensions as the rigid holder sheet 2.

As shown in Figure 1, a label 5 is preferably affixed to or printed directly on the flexible cover 1. Figure 3 shows the diagnostic device with the flexible cover partially removed, opening the container 3, thereby allowing a sample of gastric material 6 to be placed in the test composition 4 using a suitable device, such as a forceps 7.

The flexible cover 1 is affixed to the rigid container/holder material 2 by a means allowing its removal, and opening and closing of the container, such as by an adhesive which remains functional (tacky) throughout repeated openings and closings of the container.

Preferably the cover material is sufficiently thin so as to allow piercing by a syringe and injection of a liquid gastric material directly into the composition, without opening the cover. The diagnostic device described above enables positive results to be easily seen and the device is flat enough to be carried in the physician's pocket or attached to a patient's endoscopy report which enables the test to be examined for late positive results.

The following non-limiting examples illustrate the compositions, methods and devices of the present invention.

## EXAMPLE 1

A composition, according to this invention, was made comprising the following components in water :

| Component | Quantity (grams) | Final Concentration |
|---|---|---|
| urea | 3.000 | 30 g/l |
| phenol red* | 0.008 | 90 mg/l |
| methyl hydroxy benzoate | 0.200 | 2 g/l |
| bacteriological agar | 1.500 | 15 g/l |
| citric acid | 0.040 | 400 mg/l |
| sodium phosphate | 0.080 | 800 mg/l |

* : phenol sulfonphthalein indicator, having $pK_a = 7.9$, exhibiting a yellow colour in undissociated state (below pH 6.4) and red colour in dissociated state (above pH 8.2)

The components, except urea, were dissolved in 100 millilitres of water, heated to 100°C, and stirred until the solution was clear. The solution was then cooled to below 45°C, the urea added and stirred, and the solution pH was measured to be 6.0. The composition was then poured into containers, in a device of this invention, each container receiving approximately 0.3 millilitres of the composition. The composition was allowed to cool to ambient temperature, forming a gel in the container and having a deep yellow colour.

A composition made as above is used in a process of this invention, by obtaining a sample of gastric mucosa from the stomach of a human subject presenting symptoms of gastritis. The mucosa sample is then inserted into the composition and the colour observed. After approximately 15 minutes, a red colour is observed. indicating the presence of CLO-mediated gastrointestinal disorder.

## EXAMPLE II

A composition is made, according to the present invention, comprising the following components in water :

| Component | Quantity (grams) | Final Concentration |
|---|---|---|
| urea | 2.000 | 20 g/l |
| phenol red | 0.006 | 60 mg/l |
| sodium azide | 0.100 | 1 g/l |
| agar | 2.000 | 20 g/l |

The components are dissolved in 100 ml water, heated to 100°C, and the pH adjusted to pH 5.50. Approximately 0.3 millilitres of the composition is poured into the container of a device of this invention and allowed to cool, forming a gel.

The composition and device are used in a process of this invention by obtaining a sample of vomitus from a human infant subject suspected of having gastritis. A sample of the vomitus is drawn into a syringe, and a portion injected into a pH-test composition comprising 0.6 milligrams of phenol red in 10 ml of water, adjusted to pH 5.5. The colour of the pH-test composition does not change. Thereafter, the remainder of the vomitus sample is injected into the composition of this invention, by piercing the cover of the device. The colour is observed for approximately 20 minutes, noting a change in colour from deep yellow to red, and indicating the presence of gastrointestinal disorder. Modifications and variations such as would be apparent to a skilled addressee are deemed within the scope of the present invention.

## Claims

1. A composition for the diagnosis of gastrointestinal disorder in a human or lower animal subject by detection of urease in gastric material of the subject charaterised in that it comprises

(a) urea ;

(b) a bactericide which substantially inhibits growth of urease producing organisms ;

(c) a pH indicator which undergoes a colour change upon an increase of pH, at an effective concentration ; and

(d) water ;

wherein said composition has an acid pH of at least 5.0 and the pH of said composition is at least about one pH unit lower than the pKa of said indicator.

2. An composition according to claim 1, characterised in that it contains urea at a concentration of from about 10 to about 40 grams per litre.

3. A composition according to claim 2, characterised in that it contains urea at a concentration of from about 20 to about 40 grams per litre.

4. A composition according to any one of the preceding claims, characterised in that the bactericide is present at a concentration of from about 1 to about 5 grams per litre.

5. A composition according to claim 4, characterised in that the bactericide is present at a concentration of from about 2 to about 4 grams per litre.

6. A composition according to any one of the preceding claims, characterised in that the pH indicator has a $pK_a$ of from about 6.5 to about 8.5.

7. A composition according to claim 6, characterised in that the pH indicator has a $pK_a$ of from about 7.0 to about 8.0.

8. A composition according to any one of the preceding claims, characterised in that the pH indicator is present at a concentration of from about 2 to about 150 milligrams per litre.

9. A composition according to claim 8, characterised in that the pH indicator is present at a concentration of from about 20 to about 150 milligrams per litre.

10. A composition according to any one of the preceding claims, characterised in that the pH indicator is phenol red.

11. A composition according to any one of the preceding claims, characterised in that it has a pH of from about 5.0 to about 6.5.

12. A composition according to claim 11, characterised in that it has a pH of from about 5.0 to about 6.0.

13. A composition according to any one of the preceding claims, characterised in that it additionally comprises a pH buffer.

14. A composition according to any one of the preceding claims, characterised in that it additionally comprises a gelling agent.

15. A composition according to claim 14, characterised in that the gelling agent is present at a concentration of from about 5 to 50 grams per litre.

16. A composition according to claim 15, characterised in that the gelling agent is present at a concentration of from about 10 to 20 grams per litre.

17. A composition according to claim 14, 15 or 16, characterised in that the gelling agent is agar.

18. A composition according to any one of the preceding claims, characterised in that it is free from microbial nutrients.

19. A method, for the diagnosis of a gastrointestinal disorder in a human or lower animal subject by detection of urease in gastric material of the subject, characterised in that it comprises the steps of :

(a) obtaining a sample of gastric material from said subject ;

(b) contacting said sample with a composition of any one of claims 1 to 18 ; and

(c) observing the colour of said composition ; wherein a change in colour of said composition indicates the existence of a gastrointestinal disorder in said subject.

20. A device, for use in the diagnosis of gastrointestinal disorder in a human or lower animal subject by detection of urease in gastric material of the subject, characterised in that it comprises :

(a) a container, having an opening aperture area of from about 20 square millimetres to about 200 square millimetres and having a total contained volume of from about 40 cubic millimetres to about 1000 cubic millimetres ; and

(b) a cover for said container, affixed to said container by a means which allows said cover to be moved so as to open and close said container opening ; wherein said container contains from about 0.04 to

7

EP 0 204 438 B1

about 2.0 millilitres of a composition of any one of claims 1 to 18.

21. A device, according to claim 20, characterised in that said container contains from about 0.20 millitres to about 0.40 millilitres of a composition of any one of claims 1 to 18.

**Ansprüche**

1. Zusammensetzung für die Diagnose von gastrointestinalen Störungen beim Menschen oder Tier durch Bestimmung der Urease im Magenmaterial des Betroffenen, dadurch gekennzeichnet, daß sie aufweist :

(a) Harnstoff ;

(b) ein Bakterizid, das das Wachstum Urease produzierender Organismen stark hindert ;

(c) einen pH-Indikator, der bei Ansteigen des pH-Wertes einen Farbumschlag zeigt, in wirksamer Konzentration ; und

(d) Wasser ;

wobei die Zusammensetzung einen sauren pH-Wert von mindestens 5,0 besitzt und der pH-Wert der Zusammensetzung mindestens eine pH-Einheit unterhalb des pKa des Indikators liegt.

2. Zusammensetzung nach Anspruch 1, dadurch gekennzeichnet, daß sie Harnstoff in einer Konzentration von zwischen 10 bis etwa 40 Gramm pro Liter enthält.

3. Zusammensetzung nach Anspruch 2, dadurch gekennzeichnet, daß sie Harnstoff in einer Konzentration von etwa 20 bis etwa 40 Gramm pro Liter enthält.

4. Zusammensetzung nach irgendeinem der vorangehenden Ansprüche, dadurch gekennzeichnet, daß das Bakterizid in einer Konzentration von etwa 1 bis etwa 5 Gramm pro Liter vorliegt.

5. Zusammensetzung nach Anspruch 4, dadurch gekennzeichnet, daß das Bakterizid in einer Konzentration von etwa 2 bis etwa 4 Gramm pro Liter vorliegt.

6. Zusammensetzung nach irgendeinem der vorangehenden Ansprüche, dadurch gekennzeichnet, daß der pH-Indikator einen pKa-Wert von etwa 6,5 bis etwa 8,5 besitzt.

7. Zusammensetzung nach Anspruch 6, dadurch gekennzeichnet, daß der pH-Indikator einen pka-Wert von etwa 7,0 bis etwa 8,0 besitzt.

8. Zusammensetzung nach irgendeinem der vorangehenden Ansprüche, dadurch gekennzeichnet, daß der pH-Indikator in einer Konzentration von etwa 2 bis etwa 150 Milligramm pro Liter vorliegt.

9. Zusammensetzung nach Anspruch 8, dadurch gekennzeichnet, daß der pH-Indikator in einer Konzentration von etwa 20 bis etwa 150 Milligramm pro Liter vorliegt.

10. Zusammensetzung nach irgendeinem der vorangehenden Ansprüche, dadurch gekennzeichnet, daß der pH-Indikator Phenolrot ist.

11. Zusammensetzung nach irgendeinem der vorangehenden Ansprüche, dadurch gekennzeichnet, daß sie einen pH-Wert von etwa 5,0 bis etwa 6,5 aufweist.

12. Zusammensetzung nach Anspruch 11, dadurch gekennzeichnet, daß sie einem pH-Wert von etwa 5 bis etwa 6,0 aufweist.

13. Zusammensetzung nach irgendeinem der vorangehenden Ansprüche, dadurch gekennzeichnet, daß sie zusätzlich einen pH-Puffer aufweist.

14. Zusammensetzung nach irgendeinem der vorangehenden Ansprüche, dadurch gekennzeichnet, daß sie zusätzlich ein Geliermittel aufweist.

15. Zusammensetzung nach Anspruch 14, dadurch gekennzeichnet, daß das Geliermittel in einer Konzentration von etwa 5 bis 50 Gramm pro Liter vorliegt.

16. Zusammensetzung nach Anspruch 15, dadurch gekennzeichnet, daß das Geliermittel in einer Konzentration von etwa 10 bis 20 Gramm pro Liter vorliegt.

17. Zusammensetzung nach Anspruch 14, 15 oder 16, dadurch gekennzeichnet, daß das Geliermittel Agar ist.

18. Zusammensetzung nach irgendeinem der vorangehenden Ansprüche, dadurch gekennzeichnet, daß sie frei von Mikroben-Nährstoffen ist.

19. Verfahren zur Diagnose gastrointestinaler Störungen bei Mensch oder Tier durch Bestimmung der Urease im Magenmaterial des Betroffenen, dadurch gekennzeichnet, daß es die Schritte aufweist :

(a) Nehmen einer Probe von Magenmaterial des Betroffenen ;

(b) Kontaktieren der Probe mit einer Zusammensetzung nach irgendeinem der Ansprüche 1 bis 18 ; und

(c) Beobachten der Farbe der Zusammensetzung, wobei ein Farbumschlag der Zusammensetzung das Vorliegen einer gastrointestinalen Störung beim Betroffenen anzeigt.

8

20. Vorrichtung zur Verwendung bei der Diagnose von gastrointestinalen Störungen bei Menschen oder Tieren durch Bestimmung der Urease im Magenmaterial des Betroffenen, dadurch gekennzeichnet, daß sie aufweist :

(a) einen Behälter mit einer Öffnung mit einer Fläche von etwa 20 Quadratmillimeter bis etwa 200 Quadratmillimeter mit einem Gesamtvolumen von etwa 40 Kubikmillimetern bis etwa 1000 Kubikmillimetern ; und

(b) einem Deckel für den Behälter, der am Behälter mittels einer Einrichtung befestigt ist, die eine Bewegung des Deckels zur Öffnung und zum Schließen der Behälteröffnung erlaubt ; wobei der Behälter zwischen etwa 0,04 bis etwa 2,0 Milliliter einer Zusammensetzung nach irgendeinem der Ansprüche 1 bis 18 enthält.

21. Vorrichtung nach Anspruch 20, dadurch gekennzeichnet, daß der Behälter von etwa 0,20 Milliliter bis zu etwa 0,40 Milliliter einer Zusammensetzung nach irgendeinem der Ansprüche 1 bis 18 enthält.

## Revendications

1. Une composition pour le diagnostic de troubles gastro-intestinaux chez un sujet humain ou animal inférieur par détection de l'uréase dans les substances gastriques du sujet, caractérisée en ce qu'elle comprend :

a) de l'urée

b) un bactéricide qui inhibe substantiellement la croissance des organismes produisant de l'uréase ;

c) un indicateur de pH qui subit à une concentration efficace un changement de couleur lors d'une augmentation de pH ;

d) de l'eau ;

dans laquelle ladite composition a un pH acide d'au moins 5,0 et le pH de ladite composition est inférieur d'au moins environ une unité de pH au pKa dudit indicateur.

2. Une composition selon la revendication 1, caractérisée en ce qu'elle contient de l'urée à une concentration allant d'environ 10 à environ 40 grammes par litre.

3. Une composition selon la revendication 2, caractérisée en ce qu'elle contient de l'urée à une concentration allant d'environ 20 à environ 40 grammes par litre.

4. Une composition selon l'une quelconque des revendications précédentes, caractérisée en ce que le bactéricide est présent à une concentration allant d'environ 1 à environ 5 grammes par litre.

5. Une composition selon la revendication 4, caractérisée en ce que le bactéricide est présent à une concentration allant d'environ 2 à environ 4 grammes par litre.

6. Une composition selon l'une quelconque des revendications précédentes, caractérisée en ce que l'indicateur de pH a un pKa allant d'environ 6,5 à environ 8,5.

7. Une composition selon la revendication 6, caractérisée en ce que l'indicateur de pH a un pKa allant d'environ 7,0 à environ 8,0.

8. Une composition selon l'une quelconque des revendications précédentes, caractérisée en ce que l'indicateur de pH est présent à une concentration allant d'environ 2 à environ 150 milligrammes par litre.

9. Une composition selon la revendication 8, caractérisée en ce que l'indicateur de pH est présent à une concentration allant d'environ 20 à environ 150 milligrammes par litre.

10. Une composition selon l'une quelconque des revendications précédentes, caractérisée en ce que l'indicateur de pH est du rouge au phénol.

11. Une composition selon l'une quelconque des revendications précédentes, caractérisée en ce qu'elle a un pH allant d'environ 5,0 à environ 6,5.

12. Une composition selon la revendication 11, caractérisée en ce qu'elle a un pH allant d'environ 5,0 à 6,0.

13. Une composition selon l'une quelconque des revendications précédentes, caractérisée en ce qu'elle comprend additionnellement un tampon de pH.

14. Une composition selon l'une quelconque des revendications précédentes, caractérisée en ce qu'elle comprend additionnellement un agent gélifiant.

15. Une composition selon la revendication 14, caractérisée en ce que l'agent gélifiant est présent à une concentration allant d'environ 5 à 50 grammes par litre.

16. Une composition selon la revendication 15, caractérisée en ce que l'agent gélifiant est présent à une concentration allant d'environ 10 à 20 grammes par litre.

17. Une composition selon la revendication 14, 15 ou 16, caractérisée en ce que l'agent gélifiant est de l'agar-agar.

18. Une composition selon l'une quelconque des revendications précédentes, caractérisée en ce qu'elle est exempte de substances nutritives microbiennes.

19. Un procédé pour le diagnostic d'un trouble gastrointestinal chez un sujet humain ou animal inférieur par détection de l'uréase dans les substances gastriques du sujet, caractérisé en ce qu'il comprend les étapes consistant à :

a) obtenir un échantillon de substances gastriques du sujet ;

b) mettre ledit échantillon en contact avec une composition de l'une quelconque des revendications 1 à 18, et

c) observer la couleur de ladite composition ; dans lequel un changement de couleur de ladite composition indique un trouble gastro-intestinal chez ledit sujet.

20. Un dispositif utilisable pour le diagnostic de troubles gastro-intestinaux chez un sujet humain ou animal inférieur par détection de l'uréase dans les substances gastriques du sujet, caractérisé en ce qu'il comprend :

a) un récipient ayant une surface d'ouverture d'entrée allant d'environ 20 millimètres carrés à environ 200 millimètres carrés et ayant une capacité volumique totale allant d'environ 40 millimètres cubes à environ 1000 millimètres cubes ; et

b) un couvercle pour ledit récipient, fixé sur ledit récipient par un moyen qui permet audit couvercle d'être déplacé de manière à ouvrir et fermer ladite ouverture du récipient ; dans lequel ledit récipient contient d'environ 0,04 à environ 2,0 millilitres d'une composition d'une quelconque des revendications 1 à 18.

21. Un dispositif selon la revendication 20, caractérisé en ce que ledit récipient contient d'environ 0,20 millilitres à environ 0,40 millilitres d'une composition d'une quelconque des revendications 1 à 18.

FIG.1.

FIG.2

FIG.3.